# EUROPEAN PATENT APPLICATION

(11) **EP 4 527 997 A1**
(43) Date of publication of application: **26.03.2025**
(21) Application number: 23860220.5
(22) Date of filing: 25.08.2023
(51) Int. Cl.: D01F 6/14, C08F 8/00, A61F 2/08

(54) **FIBROUS POLYMER MATERIAL AND FIBROUS GEL MATERIAL**

(30) Priority: 01.09.2022 JP 2022139476
(71) Applicant: The University of Tokyo, Bunkyo-ku, Tokyo 113-8654 (JP); Gellycle Co., Ltd., Tokyo 113-0033 (JP)
(72) Inventor: SAKAI Takamasa, Tokyo 113-8654 (JP); MASUI Kosuke, Tokyo 113-0033 (JP); KAMATA Hiroyuki, Tokyo 113-0033 (JP)
(74) Representative: Symbiosis IP Limited
(86) International application number: PCT/JP2023/030771
(87) International publication number: WO 2024/048458

(57) **Abstract**

An object of the present invention is to provide a novel gel having a fibrous shape and being excellent in physical properties such as toughness and elastic modulus, and a fibrous polymer material for obtaining such a gel.

A fibrous polymer material having anisotropic swellability in water, including : a network structure in which a crystalline polymer containing a polyvinyl alcohol skeleton in a main chain is crosslinked, wherein the fibrous polymer material simultaneously exhibits following a) to c) in an equilibrium swelling state in water, and simultaneously exhibits following d) and e) from a dry state (static crystal stabilized state) to an equilibrium swelling state in water.
a) an orientation of 80% or more, the orientation as measured by wide-angle X-ray scattering (WAXS)
b) a water content of 20 to 70%
c) a crystallinity of 5 to 30%
d) a deformation rate of 75 to 120% in a long axis direction of a fibrous structure
e) a deformation rate of 120 to 200% in a short axis direction of a fibrous structure

## Description

### Technical Field

The present invention relates to a fibrous polymer material having anisotropic swellability, and a highly tough fibrous gel material obtained from the polymer material.

### Background Art

In recent years, environmental destruction and ecosystem destruction caused by plastic have become major problems. If a water-containing material in which a part of the plastic material is replaced with water can be developed, it can be a means for reducing the amount of plastic flowing out to the ocean. In particular, if a water-containing polymer material having a fibrous shape can be realized, it can be applied to a wide range of applications as a substitute material for the current plastic materials. However, in general, a polymer material having water content has a problem that toughness is not sufficient.

A polymer gel has attracted attention as a biomaterial because it exhibits high biocompatibility. However, when the water content increases, the polymer fraction as a skeleton decreases, so that the polymer gel becomes brittle and its mechanical strength decreases. Therefore, it is necessary to reduce the water content of the gel in order to obtain high mechanical strength. However, since the water content of a gel depends on a polymer forming the gel, it is difficult to arbitrarily control the water content. In addition, when a gel is formed from a polymer in a dry state involving a large size change due to expansion, the structure based on the gel may collapse. Therefore, a gel material having sufficient toughness while having a fibrous shape has not yet been put to practical use.

On the other hand, polyvinyl alcohol (PVA) is a polymer having biodegradability, and is used as a component of a high-strength fiber and a highly oriented polarizing film (for example, Non Patent Literature 1). In addition, it has been known that polyvinyl alcohol is a material whose polymer chain assembly is crystallized by hydrogen bonding when formed into a gel (for example, Non Patent Literature 2). However, a fibrous PVA gel having sufficient toughness and elastic modulus has not yet been developed.

### Citation List

### Non Patent Literature

Non Patent Literature 1: K. Ye et al., Polymer, 212, 123297, 2021
Non Patent Literature 2: J. L. Holloway et al., Soft Matter, 9, 826, 2013

### Summary of Invention

### Technical Problem

Therefore, an object of the present invention is to provide a novel gel having a fibrous shape and being excellent in physical properties such as toughness and elastic modulus, and a fibrous polymer material for obtaining such a gel.

### Solution to Problem

As a result of intensive studies to solve the above problems, the present inventors have found that when a polymer unit in which a crystalline polymer having a polyvinyl alcohol (PVA) skeleton is crosslinked is subjected to a step of swelling and drying under stretching conditions, it is possible to obtain a fibrous polymer material having different swellability (anisotropic swellability) in the long axis direction and the short axis direction of the fibrous structure under an equilibrium swelling state. In addition, the present inventors have found that when the polymer material is gelled, it is possible to obtain a fibrous gel having a specific orientation, and having unconventional excellent properties such as toughness and elasticity in spite of having a high water content. Based on these findings, the present invention has been completed.

That is, the present invention provides, in an embodiment,
<1> A fibrous polymer material having anisotropic swellability in water, including:
   a network structure in which a crystalline polymer containing a polyvinyl alcohol skeleton in a main chain is crosslinked, wherein
   the fibrous polymer material simultaneously exhibits following a) to c) in an equilibrium swelling state in water:
      a) an orientation of 80% or more, the orientation as measured by wide-angle X-ray scattering (WAXS);
      b) a water content of 20 to 70%; and
      c) a crystallinity of 5 to 30%, and
   the fibrous polymer material simultaneously exhibits following d) and e) from a dry state (static crystal stabilized state) to an equilibrium swelling state in water:
      d) a deformation rate of 75 to 120% in a long axis direction of a fibrous structure; and
      e) a deformation rate of 120 to 200% in a short axis direction of a fibrous structure;
<2> The fibrous polymer material according to <1>, wherein the water content is 30 to 60%;
<3> The fibrous polymer material according to <1>, wherein the crystalline polymer has a weight average molecular weight (Mw) of 20000 to 200000;
<4> The fibrous polymer material according to <1>, wherein the crystalline polymer has a crosslink density of 5 to 50 mmol/L;
<5> The fibrous polymer material according to <1>, having a length of 50 mm or more in a long axis direction of a fibrous structure, and a length of 5 mm or less in a short axis direction of a fibrous structure;
<6> The fibrous polymer material according to <1>, further including: a water-soluble polymer having a reactive functional group or a reactive chemical structure that reacts with an OH group of the crystalline polymer;
<7> The fibrous polymer material according to <6>, wherein the water-soluble polymer is a polyalkylene glycol;
<8> The fibrous polymer material according to <6>, wherein the reactive functional group is any of an aldehyde group, an epoxy group, and a vinyl sulfone group, and the reactive chemical structure is any of an alkenyl succinic anhydride (ASA), an acid chloride, and an acid anhydride;
<9> The fibrous polymer material according to <6>, wherein the water-soluble polymer is contained in an amount of 1 to 15% in terms of weight ratio with respect to the crystalline polymer.

In another embodiment, the present invention provides:
<10> A fibrous gel material including: the fibrous polymer material according to any one of <1> to <9>; and a solvent;
<11> The fibrous gel material according to <10>, wherein the solvent is water;
<12> The fibrous gel material according to <10>, having a polymer concentration of 30 to 80 wt%;
<13> The fibrous gel material according to <10>, having a tensile strength at break in a range of 50 to 500 MPa in a long axis direction of the fibrous structure;
<14> The fibrous gel material according to <10>, having an elastic modulus in a range of 10 to 200 MPa in a long axis direction of the fibrous structure;
<15> The fibrous gel material according to <10>, having a hysteresis loss of 1 to 25%, the hysteresis loss being obtained in a cyclic tensile test in a long axis direction of the fibrous structure, and having a maximum stress (σ) of 2 MPa or more in the test;
<16> The fibrous gel material according to <10>, having a elongation rate at break in a range of 1.2 to 3 times in a long axis direction of the fibrous structure;
<17> A three-dimensional structure including the fibrous gel material according to <10>.

### Advantageous Effects of Invention

According to the present invention, it is possible to obtain a fibrous gel material having a specific orientation, and having unconventional excellent properties such as toughness and elasticity in spite of having a high water content. In addition, the gel material is formed of a polymer having a polyvinyl alcohol skeleton with biodegradability and biocompatibility, and therefore, is expected to be a substitute for conventional plastic materials as an environmentally friendly and tough material.

Furthermore, the fibrous gel material of the present invention has a small swelling (deformation rate) in the long axis direction of the fibrous structure under an equilibrium swelling state. Therefore, even when a three-dimensional structure having a structure like knitting is constructed, the shape of the structure can be appropriately maintained. In particular, the gel has excellent hysteresis loss for elastic modulus, and also has a property of not easily breaking even when stress is repeatedly applied for a long period of time. Therefore, the gel can be applied as an artificial ligament or an artificial tendon as a fibrous biocompatible gel material.

### Brief Description of Drawings

Fig. 1 is a graph showing measurement results of a tensile test for a fibrous gel of the present invention.
Fig. 2 is a graph showing a comparison of results of a tensile test between a fibrous gel of the present invention and a rat ligament (MCL).

### Description of Embodiments

Hereinafter, embodiments of the present invention will be described. The scope of the present invention is not limited to these descriptions, and other than the following examples, the present invention can be appropriately changed and implemented without impairing the gist of the present invention.

### 1. Fibrous Polymer Material of Present Invention

The polymer material of the present invention has a fibrous shape and has anisotropic swellability in water, includes a network structure in which a crystalline polymer containing a polyvinyl alcohol skeleton in a main chain is crosslinked, and satisfies the following properties.
a) In an equilibrium swelling state in water, an orientation of 80% or more, the orientation as measured by wide-angle X-ray scattering (WAXS), is exhibited;
b) In an equilibrium swelling state in water, a water content of 20 to 70% is exhibited;
c) In an equilibrium swelling state in water, a crystallinity of 10 to 50% is exhibited;
d) From a dry state (static crystal stabilized state) to an equilibrium swelling state in water, the deformation rate is 80 to 120% in the long axis direction of the fibrous structure; and
e) From a dry state (static crystal stabilized state) to an equilibrium swelling state in water, the deformation rate is 150 to 300% in the short axis direction of the fibrous structure.

As described above, the polymer material of the present invention has a network structure in which a crystalline polymer containing a polyvinyl alcohol skeleton in a main chain is crosslinked, preferably a three-dimensional network structure. The "crystalline polymer" means a polymer having a crystal region in which polymers are regularly arranged and having a melting point. Polyvinyl alcohol (PVA) has an OH group repeating structure, and it is known that the polymer chain assembly is crystallized by hydrogen bonding. The crystalline polymer in the present invention typically preferably has a main chain only having polyvinyl alcohol, but may be a copolymer containing any other monomer components. Examples of such a monomer component include vinyl acetate, vinyl ether, and hydroxyethyl vinyl ether. The vinyl alcohol repeating unit in the crystalline polymer is preferably 90 mol% or more.

The crystalline polymer in the present invention preferably has a weight average molecular weight (Mw) in the range of 2 x 10⁴ to 2 x 10⁵, more preferably in the range of 5 x 10⁴ to 1 × 10⁵.

In a preferred embodiment, the polymer material of the present invention may further include, as a crosslinking agent for forming crosslinking between the crystalline polymers, a water-soluble polymer having a reactive functional group or a reactive chemical structure that reacts with an OH group of the crystalline polymer. Examples of the water-soluble polymer include a polyalkylene glycol, and polyethylene glycol (PEG) can be preferably used. The molecular weight of the water-soluble polymer is not particularly limited, but is typically in the range of 1 × 10³ to 1 × 10⁴.

The reactive functional group or the reactive chemical structure in the water-soluble polymer is not particularly limited as long as it can react with the OH group of the crystalline polymer to form crosslinking. The reactive functional group or the reactive chemical structure is preferably selected from the group consisting of an aldehyde group, an epoxy group, a vinyl sulfone group, alkenyl succinic anhydride (ASA), an acid chloride, an acid anhydride, and a combination thereof. Particularly preferably, the reactive functional group is an aldehyde group.

The content of the water-soluble polymer is in the range of 5 to 20%, preferably 10 to 15%, in terms of weight ratio with respect to the crystalline polymer.

In a preferred embodiment, the crystalline polymer in the present invention has a crosslink density in the range of 5 to 50 mmol/L, preferably 10 to 40 mmol/L. The crosslink density can be controlled by, for example, the content of the water-soluble polymer, the type of the reactive functional group, and the like.

As described above, the polymer material of the present invention has a fibrous shape in which the size in the long axis direction is larger than that in the short axis direction. For example, the length in the long axis direction (fiber length) is 10 times or more, preferably 100 times or more, and more preferably 1000 times or more the length in the short axis direction (fiber thickness). Specifically, the length in the long axis direction of the fibrous structure may be 50 mm or more, and the length in the short axis direction of the fibrous structure may be 5 mm or less.

Hereinafter, the characteristics of the a) to e) will be described.

The orientation of the a) can be calculated as a parameter "(180 - half width of main peak)/180 x 100" obtained by impregnating the polymer material in water and measuring by wide-angle X-ray scattering (WAXS) in an equilibrium swelling state. The orientation obtained by such a method can be 80% or more, and preferably 85% or more.

In the present specification, the "equilibrium swelling state" means a state in which the change in swelling degree reaches an equilibrium state over time during gel formation. The swelling degree can be measured by a method commonly used in the art, and the swelling degree can be a value measured at 25°C.

In the present specification, the "elastic modulus" means "Young's modulus" unless otherwise specified. The elastic modulus can be measured by a method commonly used in the art, such as a tensile test or a compression test.

In the b), the water content is a value when the polymer material is impregnated in water and brought into an equilibrium swelling state, that is, an index indicating how much water can be taken in. The water content can be 20 to 70%, preferably 25 to 65%, and more preferably 30 to 60%. As described later, the fibrous gel formed from the polymer material of the present invention is characterized by having excellent toughness and elastic modulus in spite of having such a high water content, which is a characteristic that has not been realized by conventional polymer gel materials.

In the c), the crystallinity indicates that the polymer material maintains its crystal even in an equilibrium swelling state in water, and thus the gel strength can be improved. The crystallinity can be determined as follows: in the relationship diagram between the scattering intensity and the wave number vector obtained in the WAXS measurement, the area of the region related to the peaks derived from the crystal is divided by the entire area. Specifically, the crystallinity is in the range of 5 to 40%, preferably 5 to 25. The crystallinity can be controlled by performing a swelling/drying step under stretching conditions as shown in examples described later.

In the d) and e), the deformation rate defines that when the polymer material of the present invention swells, the dimensional change is different between the length in the long axis direction (fiber length) and the length in the short axis direction (fiber thickness), that is, anisotropic swellability is exhibited. In particular, the deformation rate in the length in the long axis direction (fiber length) is smaller than in the length in the short axis direction (fiber thickness) before and after swelling. This provides an advantage that even when a three-dimensional structure having a structure like knitting is constructed using a fibrous gel, the shape of the structure can be appropriately maintained. More specifically, from a dry state (static crystal stabilized state) to an equilibrium swelling state in water, the deformation rate is 80 to 120%, preferably 85 to 110%, in the long axis direction of the fibrous structure, whereas the deformation rate is 150 to 300%, preferably 180 to 200%, in the short axis direction of the fibrous structure. Here, the deformation rate of "100%" means that the length does not change before and after swelling.

### 2. Fibrous Gel Material of Present Invention

In another embodiment, the present invention also relates to a fibrous gel material containing the above polymer material and a solvent. The fibrous gel material preferably has biocompatibility. As described above, the fibrous gel material has characteristics such as excellent toughness and elastic modulus while having a high water content.

In the present specification, the "gel" is generally a dispersion system of a polymer having high viscosity and losing fluidity, and refers to a state where the storage elastic modulus G' and the loss elastic modulus G" satisfy a relationship of G' ≥ G". The "hydrogel" is a gel containing water.

As the solvent contained in the fibrous gel material of the present invention, water or an organic solvent can be used. As the organic solvent, alcohols such as ethanol and polar solvents such as DMSO can be used. Preferably, the solvent is water. When water is used as the solvent, the fibrous gel is a hydrogel.

The fibrous gel material of the present invention preferably has a polymer concentration in the range of 30 to 80 wt%, more preferably 35 to 75 wt%.

The fibrous gel material of the present invention has a tensile strength at break of preferably 50 to 500 MPa, more preferably 75 to 400 MPa, in the long axis direction of the fibrous structure.

The fibrous gel material of the present invention has an elastic modulus of preferably 10 to 200 MPa, more preferably 30 to 180 MPa, in the long axis direction of the fibrous structure. The elastic modulus can be controlled by conditions for heating and stretching in the production process described later.

The fibrous gel material of the present invention has a hysteresis loss of preferably 1 to 20%, more preferably 1.5 to 20%, the hysteresis loss being obtained in a cyclic tensile test in the long axis direction of the fibrous structure. In the present invention, the hysteresis loss is obtained when a cyclic tensile test is performed under the condition of 10% tensile strain (ε) in the long axis direction of the fibrous structure. Specifically, the hysteresis loss can be calculated as follows: the difference between the initial area in a stress-strain curve and the second or subsequent area is divided by the initial area.

The fibrous gel material of the present invention has a elongation rate at break in the range of preferably 1.2 to 3 times, more preferably 1.2 to 2 times, in the long axis direction of the fibrous structure.

Due to these physical properties, the fibrous gel material of the present invention has excellent toughness and elasticity while having a high water content, and thus can be applied to a wide range of fields as an alternative to conventional plastic materials. In particular, the gel has excellent hysteresis loss, and also has a property that the gel is not easily broken even when stress is repeatedly applied for a long period of time. Therefore, the gel can be applied as an artificial ligament or an artificial tendon as a fibrous biocompatible gel material.

Therefore, the present invention also relates to a three-dimensional structure including the fibrous gel material. The three-dimensional structure is not particularly limited, and can be used for structures having various shapes. In particular, products to which biodegradability is desired are suitable.

### 3. Production Process of Fibrous Polymer Material and Gel of Present Invention

The fibrous polymer material of the present invention can be produced under appropriate conditions and the like by a person skilled in the art with reference to examples described later. Roughly speaking, a gel containing a crystalline polymer in a low concentration is prepared, and the gel is swollen in a solution that dissolves the crystal to be an equilibrium swelling state. Thereafter, the gel is dried and re-swollen, while the gel is stretched at a predetermined ratio. Furthermore, the gel is heated for a predetermined time in the stretching state, followed by cooling. Thereby, the fibrous polymer material of the present invention can be obtained.

Then, the fibrous polymer material is swollen in a solvent such as water. Thereby, the fibrous gel can be obtained.

### Examples

Hereinafter, the present invention will be described in more detail with reference to examples, but the present invention is not limited thereto.

### 1. Synthesis of Fibrous PVA Gel

A fibrous gel in which a PVA polymer was crosslinked was synthesized in the following procedure.

Step 1: 3 wt% of PVA having a polymerization degree of 1880 and a saponification degree of 98%, 0.443 wt% of a linear polyethylene glycol having aldehyde groups at both ends (OHC-PEG-CHO) (5000 g/mol), and 0.6 M of hydrochloric acid were dissolved in water to prepare an aqueous solution. The solution was allowed to stand overnight to prepare a gel. Thereafter, the gel was allowed to stand overnight in an environment of -20°C so that the gel was frozen.

Step 2: The frozen gel was returned to room temperature, then immersed in a 0.1 M urea solution, and allowed to stand at 50°C for 22 hours and then at 25°C for 6 hours so that the gel was swollen to an equilibrium state.

Step 3: The gel was removed from the urea solution, stretched, and dried overnight. The dried product was immersed in pure water overnight while stretched at a predetermined ratio to remove urea in the gel.

Step 4: The gel was stretched at a predetermined ratio, heated at 200°C for a predetermined time in the stretching state, and cooled at room temperature to obtain a fibrous polymer. Thereafter, the fibrous polymer was swollen in pure water to obtain a fibrous gel having a fiber length (long axis) of 200 mm and a diameter (short axis) of 0.5 mm.

### 2. Physical Properties of Fibrous PVA Gel

The obtained fibrous gel was measured for elastic modulus, breaking strain, breaking strength, breaking energy, crystallinity, orientation degree, and hysteresis loss. The results are shown in Table 1. The gel was measured for water content and deformation rate. The results are shown in Table 2. In each table, for the "Sample" column, 1 to 3 shows the results of the case where the gel was once removed therefrom and measured at each stage of Steps 1 to 3 in the synthesis described above.

For the "Sample" column, 4-1 to 4-4 shows the measurement results of the case where the heating temperature and the treatment time ("Annealing conditions" in the table) were changed in the drying under high temperature in Step 4.

**[Table 1]**

| Sample | Annealing conditions | Elastic modulus [MPa] | Breaking strain [-] | Breaking strength [MPa] | Breaking energy [MJ/m³] | Crystallinity [-] | Orientation degree (WAXS) [%] | Hysteresi loss [%] |
|---|---|---|---|---|---|---|---|---|
| 1 | | 0.00254 | 4.06 | 0.0135 | 0.0164 | | | |
| 2 | | 0.00107 | 3.76 | 0.00483 | 0.00532 | | | |
| 3 | | 4.28 | 2.32 | 29.6 | 7.89 | 8.9 | 84 | |
| 4-1 | 0.7 times 8 min | 51.6 | 1.59 | 50.8 | 13.2 | | 92 | |
| 4-2 | 1.0 times 8 min | 68.4 | 1.53 | 102 | 32.6 | 12 | 94 | 15 |
| 4-3 | 1.5 times 8 min | 59.7 | 1.52 | 159 | 32.1 | 12 | 95 | |
| 4-4 | 1.0 times 4 min | 38.1 | 1.41 | 79.0 | 20.0 | 9.0 | 93 | |

**[Table 2]**

| Sample | Water content [%] | Deformation rate in long axis direction [%] | Deformation rate in short axis direction [%] |
|---|---|---|---|
| 1 | 96.6 | | |
| 2 | 97.8 | | |
| 3 | 70.6 | | |
| 4-1 | 33.1 | 106 | 146 |
| 4-2 | 39.9 | 95 | 151 |
| 4-3 | 44.9 | 83 | 136 |
| 4-4 | 46.5 | 93 | 125 |

### <Longitudinal and Vertical Deformation Rates>

For each sample shown in each table, the fiber in a dry state was swollen in a water bath at 37°C. One hour later, the length in the fiber direction and the diameter direction was measured, and compared with those before swelling to determine the fiber axis ratio and the diameter ratio.

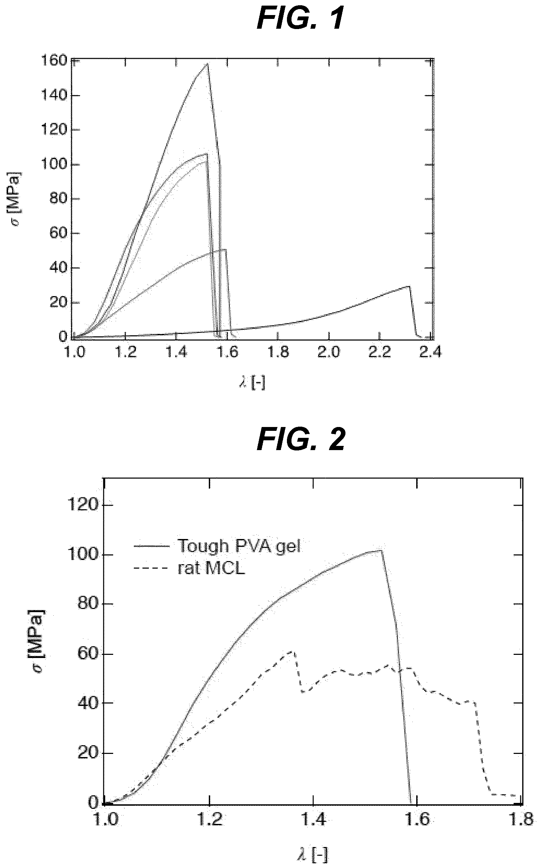

For each sample shown in each table, the measurement results of the tensile test are shown in Fig. 1. It was found that the breaking strength is significantly improved by performing the drying treatment under high temperature in Step 4. This is presumably because stretched and oriented polymer chains were crystalized through the drying treatment under high temperature, so that toughness was obtained.

In addition, Fig. 2 shows a graph indicating the results of the tensile test for the fibrous gel material of Sample 4-2 and a rat ligament (MCL) as a comparative example. The fibrous gel of the present invention exhibited a stretch rate of about 1.6 times, a breaking strength exceeding 100 MPa, and an elastic modulus of 68.4 MPa, which exceeded the strength required for an artificial ligament.

### <Result of Hysteresis Loss>

For each sample shown in each table, a predetermined strain was repeatedly applied at a cycle of 1 Hz with a tensile tester in a water bath at 37 degrees (cyclic tensile test), and the relationship between stress (σ) and strain (ε) (hysteresis loop) was obtained. From the area of the obtained hysteresis loop, the hysteresis loss between the initial time and the second or subsequent times was calculated.

The above results indicate that the fibrous gel of the present invention has excellent toughness as compared with existing gels. In addition, since the polymer concentration in the equilibrium swelling state was also a value close to that of the biological soft tissue, it was demonstrated that the fibrous gel has non-swellability and toughness.

## Claims

1. A fibrous polymer material having anisotropic swellability in water, comprising:
a network structure in which a crystalline polymer containing a polyvinyl alcohol skeleton in a main chain is crosslinked, wherein
the fibrous polymer material simultaneously exhibits following a) to c) in an equilibrium swelling state in water:
a) an orientation of 80% or more, the orientation as measured by wide-angle X-ray scattering (WAXS);
b) a water content of 20 to 70%; and
c) a crystallinity of 5 to 30%, and
the fibrous polymer material simultaneously exhibits following d) and e) from a dry state (static crystal stabilized state) to an equilibrium swelling state in water:
d) a deformation rate of 75 to 120% in a long axis direction of a fibrous structure; and
e) a deformation rate of 120 to 200% in a short axis direction of a fibrous structure.

2. The fibrous polymer material according to claim 1, wherein the water content is 30 to 60%.

3. The fibrous polymer material according to claim 1, wherein the crystalline polymer has a weight average molecular weight (Mw) of 20000 to 200000.

4. The fibrous polymer material according to claim 1, wherein the crystalline polymer has a crosslink density of 5 to 50 mmol/L.

5. The fibrous polymer material according to claim 1, having a length of 50 mm or more in a long axis direction of a fibrous structure, and a length of 5 mm or less in a short axis direction of a fibrous structure.

6. The fibrous polymer material according to claim 1, further comprising: a water-soluble polymer having a reactive functional group or a reactive chemical structure that reacts with an OH group of the crystalline polymer.

7. The fibrous polymer material according to claim 6, wherein the water-soluble polymer is a polyalkylene glycol.

8. The fibrous polymer material according to claim 6, wherein the reactive functional group is any of an aldehyde group, an epoxy group, and a vinyl sulfone group, and the reactive chemical structure is any of an alkenyl succinic anhydride (ASA), an acid chloride, and an acid anhydride.

9. The fibrous polymer material according to claim 6, wherein the water-soluble polymer is contained in an amount of 1 to 15% in terms of weight ratio with respect to the crystalline polymer.

10. A fibrous gel material comprising: the fibrous polymer material according to any one of claims 1 to 9; and a solvent.

11. The fibrous gel material according to claim 10, wherein the solvent is water.

12. The fibrous gel material according to claim 10, having a polymer concentration of 30 to 80 wt%.

13. The fibrous gel material according to claim 10, having a tensile strength at break in a range of 50 to 500 MPa in a long axis direction of the fibrous structure.

14. The fibrous gel material according to claim 10, having an elastic modulus in a range of 10 to 200 MPa in a long axis direction of the fibrous structure.

15. The fibrous gel material according to claim 10, having a hysteresis loss of 1 to 25%, the hysteresis loss being obtained in a cyclic tensile test in a long axis direction of the fibrous structure, and having a maximum stress (σ) of 2 MPa or more in the test.

16. The fibrous gel material according to claim 10, having a elongation rate at break in a range of 1.2 to 3 times in a long axis direction of the fibrous structure.

17. A three-dimensional structure comprising the fibrous gel material according to claim 10.
